# EUROPÄISCHE PATENTSCHRIFT

(11) **EP 3 409 467 B1**
(45) Veröffentlichungstag und Bekanntmachung des Hinweises auf die Patenterteilung: **03.07.2019**
(21) Anmeldenummer: 17173559.0
(22) Anmeldetag: 30.05.2017
(51) Int. Cl.: B32B 18/00, C04B 35/10, G01K 7/18, H01C 1/016, H05B 3/12, C04B 35/48, C04B 37/02

(54) **HEIZER MIT EINEM CO-GESINTERTEN MEHRSCHICHTENAUFBAU**
HEATER WITH A CO-SINTERED MULTI-LAYER STRUCTURE
DISPOSITIF DE CHAUFFAGE COMPRENANT UNE STRUCTURE MULTICOUCHES CO-FRITTÉE

(43) Veröffentlichungstag der Anmeldung: 05.12.2018
(73) Patentinhaber: Heraeus Nexensos GmbH, 63450 Hanau (DE)
(72) Erfinder: Dietmann, Stefan, 63755 Alzenau (DE); Teusch, Dieter, 63486 Bruchköbel (DE); Asmus, Tim, 35469 Allendorf-Winnen (DE); Wienand, Karlheinz, 63741 Aschaffenburg (DE)
(74) Vertreter: Heraeus IP

(56) Entgegenhaltungen:
- EP-A1- 2 316 286
- WO-A2-2010/089024
- DE-A1- 3 941 837
- DE-A1-102014 104 219
- US-A1- 2004 094 417

## Beschreibung

Die vorliegende Erfindung betrifft ein Verfahren zur Herstellung eines Heizers mit einem co-gesinterten Mehrschichtaufbau für ein System zur Bereitstellung eines inhalierbaren Aerosols. Weiterhin betrifft die vorliegende Erfindung eine Verwendung eines Heizers, ein System zur Bereitstellung eines inhalierbaren Aerosols, sowie einen Heizer.

Aus dem Stand der Technik sind Systeme mit Heizern zur Bereitstellung von inhalierbaren Aerosolen, die eine Substanz zur Bildung des Aerosols aufheizen, bekannt. Abhängig von dem verwendeten System kann die Substanz flüssig, gasförmig oder fest sein. Im Allgemeinen wird in derartigen Systemen eine Substanz, die Tabak oder ein tabakähnliches Produkt umfasst, nicht wie in einer konventionellen Zigarette verbrannt, sondern lediglich erhitzt, ohne die Substanz zu verbrennen, um dadurch ein inhalierbares Aerosol zu erhalten.

Beispielsweise beschreibt die WO 2013/034456 A1 den Aufbau eines Systems in dem Tabak oder ein tabakähnliches Produkt, das sich in einem Vorratsbehältnis in dem System befindet, an einer Oberfläche oder in der Nähe der Oberfläche eines Heizers erhitzt wird, bis ein nikotinhaltiges und/oder geschmackstoffhaltiges Aerosol entsteht. Das gebildete Aerosol kann mittels eines Mundstücks von einem Benutzer inhaliert werden.

Ein weiteres beispielhaft beschriebenes System zur Bereitstellung eines inhalierbaren Aerosols wird in der WO 2016/207407 A1 beschrieben. Das System umfasst ein Gehäuse mit einer Öffnung zum Einbringen der Substanz zur Bildung des Aerosols. In dem Gehäuse ist ein Heizer angeordnet, der die eingebrachte Substanz erhitzt, aber nicht vollständig verbrennt. Auch in diesem Beispiel kann das gebildete Aerosol mittels eines Mundstücks von dem Benutzer inhaliert werden.

Ein typischer Aufbau eines Heizers für ein System zur Bereitstellung eines inhalierbaren Aerosols wird beispielhaft in der WO 2011/050964 A1 beschrieben. Auf ein elektrisch isolierendes Substrat, wie beispielsweise einer elektrisch isolierenden Keramik, wird eine Metallpaste aufgetragen und anschließend ein Muster in die aufgetragene Metallpaste eingebracht, so dass ein Widerstandselement in Form einer Widerstandsleiterbahn entsteht.

Danach wird das Substrat mit der aufgetragenen Metallpaste in einem zweistufigen Verfahren gesintert. In einem zusätzlichen Schritt kann danach auf die Widerstandsleiterbahn eine Passivierungsschicht aufgesintert werden.

Aus dem Stand der Technik sind auch noch weitere mehrstufige Herstellungsverfahren, zum Herstellen von komplexeren Heizern bekannt. Beispielsweise beschreibt die WO 2013/017185 A1 einen Heizprägestempel mit einem wärmeisolierenden Substrat auf dem eine elektrisch leitende Struktur, umfassend einen Heizwiderstand angeordnet ist. Die elektrisch leitende Struktur ist mit einer elektrisch isolierenden Schicht abgedeckt. Beispielsweise kann der Heizprägestempel hergestellt werden mit einem Verfahren, in dem zunächst eine Isolierschicht auf ein elektrisch leitendes Substrat aufgesintert wird. Danach wird ein Widerstandselement aufgesintert, auf das in einem weiteren Ausheizschritt wiederum eine Isolierschicht aufgesintert wird.

Die aus dem Stand der Technik bekannten Verfahren haben somit den Nachteil, dass diese Verfahren eine hohe Anzahl von Verarbeitungs- und Ausheizschritten verlangen. Dadurch wird die Herstellung von Heizern für Systeme zur Bereitstellung eines inhalierbaren Aerosols sehr komplex und somit zeitaufwendig und teuer.

Aufgabe der vorliegenden Erfindung ist es daher, ein verbessertes Verfahren zur Herstellung eines Heizers bereitzustellen, das die Nachteile des Stands der Technik überwindet. Insbesondere ein Verfahren bereitzustellen, mit dem ein Heizer kostengünstig und schnell herstellbar ist.

Diese Aufgabe wird erfindungsgemäß durch ein Verfahren gemäß des Gegenstands des Patentanspruchs 1 gelöst.

Das erfindungsgemäße Verfahren zur Herstellung eines Heizers mit einem co-gesinterten Mehrschichtaufbau für ein System zur Bereitstellung eines inhalierbaren Aerosols weist hierfür auf:
Bereitstellen zumindest eine erste Substratschicht;
Anordnen zumindest eine erste Isolierschicht zumindest bereichsweise auf der ersten Substratschicht;
Anordnen zumindest eines Heizelements zumindest bereichsweise auf der ersten Isolierschicht;
Anordnen zumindest eine zweite Substratschicht und zumindest eine zweite Isolierschicht zumindest bereichsweise auf dem Heizelement, wobei die zweite Isolierschicht zumindest bereichsweise auf der zweiten Substratschicht angeordnet ist, und wobei die zweite Isolierschicht zumindest bereichsweise mit dem Heizelement und/oder der ersten Isolierschicht in Verbindung steht;
Verpressen der Schichten und des Heizelements; und
Brennen der verpressten Schichten, zum Co-Sintern der Schichten des Mehrschichtsaufbaus.

Der Heizer kann zum Erhitzen oder Verdampfen einer Substanz in einem Temperaturbereich von 200°C bis 400°C betrieben werden. Der Heizer kann aber, zumindest vorübergehend, auch in einem höheren Temperaturbereich, beispielsweise bei ungefähr 550°C zur Reinigung des Heizers betrieben werden.

Der Begriff "Mehrschichtaufbau" kann hierein verwendet werden, um einen Aufbau zu bezeichnen, der die verschiedenen Schichten des Heizers umfasst.

Unter den Begriffen "co-gesintert" und "co-gesinterter Mehrschichtaufbau" kann ein gleichzeitiges Erhitzen und Ausheizen von mehreren Schichten eines Mehrschichtaufbaus, beispielsweise unter Druck oder drucklos, bezeichnet werden, so dass die Schichten des Mehrschichtaufbaus in einem einzigen Arbeitsgang gebrannt, beziehungsweise zusammengesintert werden können. Die einzelnen Schichten können vor dem Brennen vorgetrocknet werden.

Die Schritte des erfindungsgemäßen Verfahrens können in einem Beispiel aufeinanderfolgend durchgeführt werden. In einem anderen Beispiel, können die Schritte in einer beliebigen Reihenfolge durchgeführt werden und/oder einzelne Verfahrensschritte ausgelassen, bzw. hinzugefügt werden.

Der Erfindung liegt die überraschende Erkenntnis zu Grunde, dass durch ein Co-sintern des Mehrschichtaufbaus die Anzahl der Ausheizschritte auf einen einzelnen Ausheizschritt reduziert wird und damit die Herstellungskosten des Heizers signifikant gesenkt werden können.

In einem Beispiel der Erfindung umfasst/umfassen die erste Substratschicht und/oder die zweite Substratschicht zumindest eine Grünfolie, insbesondere eine Grünfolie aufweisend stabilisiertes Zirkoniumoxid, ZrO2.

Unter dem Begriff "Grünfolie" kann eine Substratschicht und/oder eine Folie verstanden werden, die einen Feststoffanteil umfasst, der organisches Material und anorganisches Material aufweisen kann. Der organische Anteil unter den Feststoffen kann, z.B. Aluminiumoxid, Yttrium- oder Scandium-stabilisiertes Zirkoniumoxid oder auch andere keramische, metallische oder glasige Werkstoffe umfassen, zusätzlich kann der anorganische Anteil Reststoffe in entsprechender Menge umfassen. Der organische Anteil kann weiterhin Binder, Weichmacher und Dispergierer umfassen. Als Weichmacher können Phthalate, Polyole, Glykole, Phosphate sowie andere schwerflüchtige Kohlenwasserstoffe und dergleichen eingesetzt werden. Ein Herstellungsverfahren einer keramischen Grünfolie ist beispielsweise in der DE 19924134 A1 beschrieben.

Der Begriff "stabilisiertes Zirkoniumoxid" kann verwendet werden, um ein Yttrium stabilisiertem Zirconiumdioxid als erste und/oder zweite Substratschicht zu bezeichnen, wie es beispielsweise in der WO 2010/089024 A1 beschrieben ist. Die Sauerstoffleitfähigkeit der ersten und/oder zweiten Substratschicht kann durch die Zugabe von Tantal oder Niob unterdrückt werden. Anstatt Yttrium sind auch andere zwei oder dreiwertige Metalle wie z.B. Scandium zur Stabilisierung einsetzbar. Vorteilhaft kann eine hohe Bruchfestigkeit durch einen Gehalt von 20-40 Mol-% Stabilisator, bezogen auf den gesamten Metallgehalt, insbesondere 25-35 Mol-%, erreicht werden, weil hierdurch die Hochtemperatur-tetragonale Phase des Zirconiumdioxids erhalten bleibt. Die mechanische Festigkeit kann weiterhin durch Dotierung mit HfO2 auf über 250 MPa gesteigert werden.

In einem weiteren Beispiel weist die Grünfolie eine Dicke in einem Bereich zwischen 0,25 mm und 0,5 mm auf.

In einem Beispiel weist die erste Isolierschicht und/oder die zweite Isolierschicht ein Aluminiumoxid, Al2O3 auf, insbesondere einen Keramikschlicker aufweisend Aluminiumoxid, Al2O3.

Beispielsweise kann ein Keramikschlicker aufweisend Aluminiumoxid verwendet werden, wie es beispielsweise in der DE4016861 A1 beschrieben wird. Hier wird ein feinteiliges Aluminiumoxid-Pulver mit Hilfe weiterer organischer und anorganischer Additive zu einer Giessmasse verarbeitet, zur Herstellung einer Grünfolie. Vorteilhaft wirkt eine derartige Isolierschicht auch bei hohen Temperatur von über 1000°C noch isolierend. Weiterhin ist eine derartige Isolierschicht, wegen den hervorragenden mechanischen Eigenschaften, besonders gut für eine Co-sinterung geeignet. Insbesondere kann durch den Einsatz eines solchen Keramikschlickers ein im Wesentlichen rissfreies Gefüge erhalten werden.

In einem weiteren Beispiel weist das Anordnen der ersten Isolierschicht und/oder der zweiten Isolierschicht auf:
Siebdrucken und/oder Tape-Casting der ersten Isolierschicht auf der ersten Substratschicht und/oder der zweiten Isolierschicht auf der zweiten Substratschicht.

Durch Siebdrucken und/oder Tape Casting kann/können die erste Isolierschicht und/oder die zweite Isolierschicht besonders schnell und effizient angeordnet werden.

In einem Beispiel umfasst das Heizelement zumindest ein Widerstandselement, insbesondere ein Widerstandselement umfassend Platin, Pt-Cermet.

Beispielsweise kann das Heizelement als Widerstandselement in Form einer Leiterbahn ausgebildet werden. Wenn die Leiterbahn von einem Strom durchflossen wird, entsteht durch den intrinsischen Widerstand der Leiterbahn Wärme. Abhängig von dem, die Leiterbahn durchfließenden, Strom kann eine festgelegte Heizleistung des Heizers festgelegt werden.

Bevorzugt ist der Pt-Cermet wie folgt zusammengesetzt:
45% Gew% Pt-Pulver mit einer Korngröße von 50 µm,
45% Gew% Al2O3-Pulver mit einer Korngröße von 2 µm, und
10% Gew% Bindemittel, beispielsweise METAWAX P-50 der Firma Zschimmer & Schwarz GmbH & Co.KG, oder ein ähnliches Bindemittel.

Die relativen Anteile sind jeweils bezogen auf das Gesamtgewicht des PT-Cermets.

Für die Herstellung des Pt-Cermets kann das Pt-Pulver, das Al2O3-Pulver und das Bindemittel auf einem Rollenbock mit Al2O3-Kugeln gemischt werden. Danach kann die entstandene Mischung in einen Doppel-Z-Kneter gefüllt werden und unter Zugabe von demineralisiertem Wasser, VE Wasser, mit einem Dispergator weiter vermengt werden, um Lufteinschlüsse zu entfernen.

In noch einem Beispiel weist das Anordnen des Heizelements auf: Siebdrucken des Widerstandselements auf die erste Isolierschicht.

Beispielsweise kann die zuvor beschriebene Mischung unter mindestens 80% Luftfeuchtigkeit mittels eines Siebdruckverfahrens auf die Isolierschicht aufgebracht werden.

In einem Beispiel weist das Anordnen der zweiten Substratschicht und der zweiten Isolierschicht auf:
Anordnen der zweiten Isolierschicht zumindest bereichsweise auf dem Heizelement und Anordnen der zweiten Substratschicht zumindest bereichsweise auf der zweiten Isolierschicht; oder
Anordnen der zweiten Isolierschicht zumindest bereichsweise auf der zweiten Substratschicht und Anordnen der zweiten Isolierschicht zumindest bereichsweise auf dem Heizelement.

Beispielsweise kann die zweite Isolierschicht zunächst auf dem Heizelement und/oder der ersten Isolierschicht angeordnet werden, so dass die zweite Isolierschicht zumindest bereichsweise mit dem Heizelement und/oder der ersten Isolierschicht in Verbindung steht und danach kann die zweite Substratschicht auf der gegenüberliegenden Seite der zweiten Isolierschicht angeordnet werden. In einem weiteren Beispiel kann die zweite Isolierschicht zunächst auch auf der zweiten Substratschicht angeordnet werden, um danach die zweite Substratschicht mit der daran angeordneten zweiten Isolierschicht zusammen auf dem Heizelement, oder der ersten Isolierschicht anzuordnen, so dass die zweite Isolierschicht zumindest bereichsweise mit dem Heizelement und/oder der ersten Isolierschicht in Verbindung steht.

In einem weiteren Beispiel weist das Brennen der verpressten Schichten auf: Brennen der verpressten Schichten des Mehrschichtsaufbaus bei mindestens 1400°C für mindestens 36 Stunden.

Das Brennen der verpressten Schichten kann beispielsweise nach einem in der US 20040094417 A1 oder in der DE 19545590 C2 beschriebenen Verfahren erfolgen.

In einem weiteren Beispiel weist das Verfahren auf:
Vereinzeln des Heizers vor dem Brennen, insbesondere durch Ausstanzen oder durch ein Laserschneidverfahren, oder
Vereinzeln des Heizers nach dem Verpressen der Schichten und vor dem Brennen.

Vorteilhaft kann durch eine Vereinzelung des Heizers vor dem Brennen, die Entstehung von Mikrorissen in der Keramik entgegengewirkt werden.

In einem Beispiel werden Ausnehmungen in die zweite Isolierschicht und/oder in die zweite Substratschicht eingebracht, insbesondere werden Ausnehmungen eingebracht zur zumindest bereichsweisen Freilegung zumindest eines Anschlussmittels des Heizelements.

Diese Ausnehmungen dienen der Kontaktierung des Heizelements. Beispielsweise können die Ausnehmungen in Form von ausgestanzten Löchern ausgestaltet sein, durch die jeweils zumindest ein Anschlussleiter zur Kontaktierung von Anschlusskontakten an dem Heizelement hindurchgeführt werden können. Eine Ausnehmung kann aber auch als eine Verkürzung einer jeweiligen Isolier- und/oder Substratschicht verstanden werden, die es ermöglicht, dass die Anschlusskontakte von einer Außenseite des Heizers zugänglich sind.

In einem weiteren Beispiel weist das Verfahren auf:
Anordnen zumindest eines Temperatursensors, vor dem Verpressen der Schichten, zwischen:
(i) der ersten Isolierschicht und der ersten Substratschicht und/oder
(ii) der zweiten Isolierschicht und der zweiten Substratschicht und/oder
(iii) der ersten Isolierschicht und der zweiten Isolierschicht.

Vorteilhaft kann durch das integrale Anordnen eines Temperatursensors zwischen den Schichten, die Temperatur des Heizers zuverlässig ermittelt werden und dementsprechend der Stromfluss durch den Heizer gesteuert oder geregelt werden.

Auch schlägt die Erfindung eine Verwendung eines Heizers hergestellt durch ein erfindungsgemäßes Verfahren in einem System zur Bereitstellung eines inhalierbaren Aerosols vor.

Weiterhin schlägt die Erfindung ein System zur Bereitstellung eines inhalierbaren Aerosols, aufweisend zumindest einen Heizer hergestellt durch ein erfindungsgemäßes Verfahren vor.

Zudem schlägt die Erfindung einen Heizer mit einem co-gesinterten Mehrschichtaufbau für ein System zur Bereitstellung eines inhalierbaren Aerosols vor, aufweisend:
zumindest ein Heizelement mit einer ersten Seite und einer der ersten Seite gegenüberliegenden zweiten Seite;
zumindest eine erste und eine zweite Isolierschicht, wobei die erste Isolierschicht zumindest bereichsweise auf der ersten Seite des Heizelements angeordnet ist, und wobei die zweite Isolierschicht zumindest bereichsweise auf der zweiten Seite des Heizelements angeordnet ist; und
zumindest eine erste Substratschicht und eine zweite Substratschicht, wobei die erste Substratschicht zumindest bereichsweise auf der ersten Isolierschicht angeordnet ist, und wobei die zweite Substratschicht zumindest bereichsweise auf der zweiten Isolierschicht angeordnet ist.

Weitere Merkmale und Vorteile der Erfindung ergeben sich aus der nachfolgenden Beschreibung, in der bevorzugte Ausführungsformen der Erfindung anhand von schematischen Zeichnungen erläutert sind.

Dabei zeigt:
- Figur 1: eine schematische Explosionsdarstellung eines Heizers gemäß einer Ausführungsform der Erfindung; und
- Figur 2: ein Verfahren zur Herstellung eines Heizers gemäß einer Ausführungsform der Erfindung.

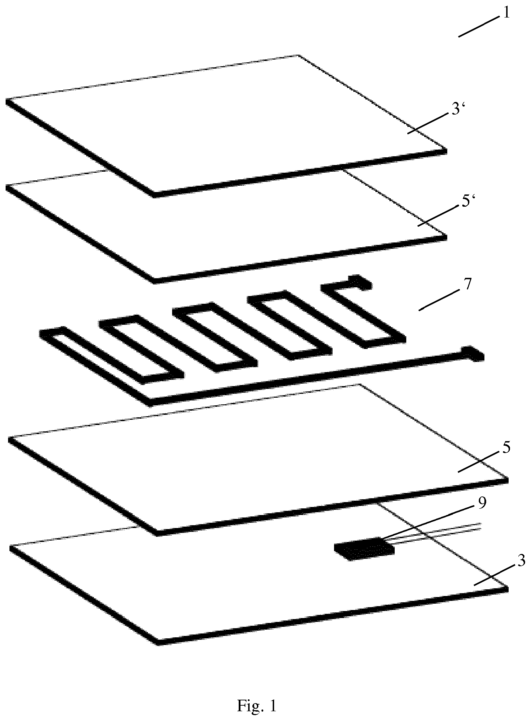

Figur 1 zeigt eine schematische Explosionsdarstellung eines Heizers 1 gemäß einer Ausführungsform der Erfindung.

Auf einer ersten Substratschicht 3, die in der gezeigten Ausführungsform eine Grünfolie, insbesondere eine Grünfolie aufweisend stabilisiertes ZrO2 sein kann, ist eine erste Isolierschicht 5 angeordnet. In der gezeigten Ausführungsform kann die erste Isolierschicht 5 einen Keramikschlicker, aufweisend Al2O3, umfassen und auf der ersten Substratschicht 3 mittels Siebdrucken und/oder Tape-Casting angeordnet werden. Nach dem Anordnen der ersten Isolierschicht 5 auf der ersten Substratschicht 3 kann der Aufbau vor einer weiteren Verarbeitung getrocknet werden.

Zwischen der ersten Substratschicht 3 und der ersten Isolierschicht 5 ist in der gezeigten Ausführungsform ein Temperatursensor 9 angeordnet. Der Temperatursensor 9 dient zur Ermittlung einer Temperatur des Heizers 1. In Figur 1 ist der Temperatursensor 9 lediglich optional gezeigt, da ein derartiger Sensor für die Erfindung nicht wesentlich ist. Auch kann/können der Temperatursensor 9 oder mehrere Temperatursensoren (nicht gezeigt) zwischen anderen Schichten des Mehrschichtaufbaus angeordnet sein. In nicht gezeigten Ausführungsformen kann der Temperatursensor auch zwischen der ersten Isolierschicht 5 und einer zweiten Isolierschicht 5' und/oder zwischen der zweiten Isolierschicht 5' und einer zweiten Substratschicht 3' angeordnet sein.

Auf der ersten Isolierschicht 5 ist in der gezeigten Ausführungsform ein Heizelement 7 angeordnet. In Figur 1 ist das Heizelement 7 als Widerstandselement in Form einer Leiterbahn realisiert. In der gezeigten Ausführungsform kann das Widerstandselement einen Pt-Cermet umfassen und mittels eines Siebdruckverfahrens auf der ersten Isolierschicht 5 angeordnet sein. In Figur 1 ist das Widerstandselement als mäanderförmig geführte Leiterbahn mit zwei Anschlusskontakten dargestellt. In nicht gezeigten Ausführungsformen kann die Leiterbahn auch anders ausgestaltet sein. Beispielsweise kann die Leiterbahn auch spiralförmig ausgestaltet sein. Das Heizelement kann, in einer nicht gezeigten Ausführungsform, durch geeignete elektronische Ansteuerung auch selbst die Funktion eines Temperatursensors übernehmen.

Auf dem Heizelement 7 ist in der gezeigten Ausführungsform die zweite Isolierschicht 5' angeordnet, die wiederum auf der zweiten Substratschicht 3' angeordnet ist. Die Materialien der ersten und zweiten Isolierschicht 5, 5' und der ersten und zweiten Substratschicht 3, 3' können im Wesentlichen gleich sein. Auch können die zweite Isolierschicht 5' und die zweite Substratschicht 3' nacheinander, in ähnlicher Weise wie auch die erste Isolierschicht 5 und die erste Substratschicht 3 angeordnet werden. Alternativ hierzu kann die zweite Isolierschicht 5' zunächst auch auf der zweiten Substratschicht 3' angeordnet werden, um danach die zweite Substratschicht 3' mit daran angeordneter zweiter Isolierschicht 5' zusammen auf dem Heizelement 7 anzuordnen.

In der gezeigten Ausführungsform sind in die zweite Isolierschicht 5' und in die zweite Substratschicht 3' Ausnehmungen in Form einer Verkürzung der zweiten Isolierschicht 5' relativ zu der ersten Isolierschicht 5 und der zweiten Substratschicht 3' relativ zu der ersten Substratschicht 3 eingebracht, zur zumindest bereichsweisen Freilegung der Anschlusskontakte des Heizelements 1. In nicht gezeigten Ausführungsformen können die Ausnehmungen auch in Form von ausgestanzten Löchern ausgestaltet sein, durch die jeweils zumindest ein Anschlussleiter zur Kontaktierung des Heizelements 7 hindurchgeführt werden kann.

Die in Figur 1 gezeigten Schichten des Mehrschichtaufbaus sind im Wesentlichen planar, bzw. eben. In nicht gezeigten Ausführungsformen können diese Schichten aber auch eine konkave oder konvexe Wölbung aufweisen, bzw. zylinderförmig gebogen sein, um die zu erhitzende Substanz zu umgeben. Zum Beispiel kann in dieser nicht gezeigten Ausführungsform die zu erhitzende Substanz in dem Zylinder angeordnet, bzw. hineingeschoben werden.

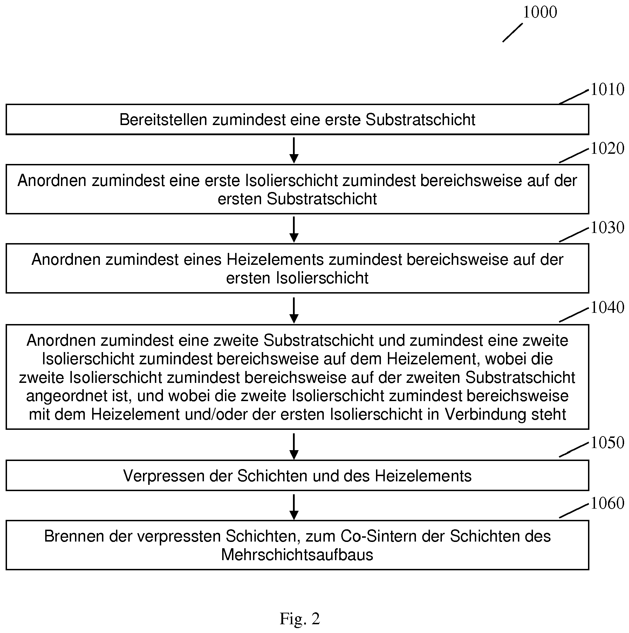

Figur 2 zeigt ein Verfahren zur Herstellung eines Heizers gemäß einer Ausführungsform der Erfindung.

Das gezeigte Verfahren zur Herstellung eines Heizers mit einem co-gesinterten Mehrschichtaufbau für ein System zur Bereitstellung eines inhalierbaren Aerosols, weist die nachfolgenden Schritte auf:
Bereitstellen 1010 zumindest eine erste Substratschicht;
Anordnen 1020 zumindest eine erste Isolierschicht zumindest bereichsweise auf der ersten Substratschicht;
Anordnen 1030 zumindest eines Heizelements zumindest bereichsweise auf der ersten Isolierschicht;
Anordnen 1040 zumindest eine zweite Substratschicht und zumindest eine zweite Isolierschicht zumindest bereichsweise auf dem Heizelement, wobei die zweite Isolierschicht zumindest bereichsweise auf der zweiten Substratschicht angeordnet ist, und wobei die zweite Isolierschicht zumindest bereichsweise mit dem Heizelement und/oder der ersten Isolierschicht in Verbindung steht;
Verpressen 1050 der Schichten und des Heizelements; und
Brennen 1060 der verpressten Schichten, zum Co-Sintern der Schichten des Mehrschichtsaufbaus.

Die in der vorstehenden Beschreibung, in den Ansprüchen und in den Figuren dargestellten Merkmale können sowohl einzeln als auch in beliebiger Kombination wesentlich für die Erfindung in ihren verschiedenen Ausführungsformen sein.

### Bezugszeichenliste

- 1: Heizer
- 3, 3': Substratschicht
- 5, 5': Isolierschicht
- 7: Heizelement
- 9: Temperatursensor

- 1010: Bereitstellen erste Substratschicht
- 1020: Anordnen erste Isolierschicht
- 1030: Anordnen Heizelement
- 1040: Anordnen zweite Substratschicht und zweite Isolierschicht
- 1050: Verpressen
- 1060: Brennen

## Patentansprüche

1. Verfahren zur Herstellung eines Heizers (1) mit einem co-gesinterten Mehrschichtaufbau für ein System zur Bereitstellung eines inhalierbaren Aerosols, aufweisend die Schritte:
Bereitstellen (1010) zumindest eine erste Substratschicht (3);
Anordnen (1020) zumindest eine erste Isolierschicht (5) zumindest bereichsweise auf der ersten Substratschicht (3);
Anordnen (1030) zumindest eines Heizelements (7) zumindest bereichsweise auf der ersten Isolierschicht (5);
Anordnen (1040) zumindest eine zweite Substratschicht (3') und zumindest eine zweite Isolierschicht (5') zumindest bereichsweise auf dem Heizelement (7), wobei die zweite Isolierschicht (5') zumindest bereichsweise auf der zweiten Substratschicht (3') angeordnet ist, und wobei die zweite Isolierschicht (5') zumindest bereichsweise mit dem Heizelement (7) und/oder der ersten Isolierschicht (5) in Verbindung steht;
Verpressen (1050) der Schichten und des Heizelements (7); und
Brennen (1060) der verpressten Schichten, zum Co-Sintern der Schichten des Mehrschichtsaufbaus.

2. Verfahren nach Anspruch 1, **dadurch gekennzeichnet, dass** die erste Substratschicht (3) und/oder die zweite Substratschicht (3') zumindest eine Grünfolie umfasst/umfassen, insbesondere eine Grünfolie aufweisend stabilisiertes Zirkoniumoxid, ZrO2.

3. Verfahren nach Anspruch 2, **dadurch gekennzeichnet, dass** die Grünfolie eine Dicke in einem Bereich zwischen 0,25 mm und 0,5 mm aufweist.

4. Verfahren nach einem der vorangehenden Ansprüche, **dadurch gekennzeichnet, dass** die erste Isolierschicht (5) und/oder die zweite Isolierschicht (5') ein Aluminiumoxid, Al2O3 aufweist, insbesondere einen Keramikschlicker aufweisend Aluminiumoxid, Al2O3.

5. Verfahren nach einem der vorangehenden Ansprüche, **dadurch gekennzeichnet, dass** das Anordnen der ersten Isolierschicht (5) und/oder der zweiten Isolierschicht (5') aufweist:
Siebdrucken und/oder Tape-Casting der ersten Isolierschicht (5) auf der ersten Substratschicht (3) und/oder der zweiten Isolierschicht (5') auf der zweiten Substratschicht (3').

6. Verfahren nach einem der vorangehenden Ansprüche, **dadurch gekennzeichnet, dass** das Heizelement (7) zumindest ein Widerstandselement umfasst, insbesondere ein Widerstandselement umfassend Platin, Pt,-Cermet.

7. Verfahren nach Anspruch 6, **dadurch gekennzeichnet, dass** das Anordnen (1030) des Heizelements (7) aufweist:
Siebdrucken des Widerstandselements auf die erste Isolierschicht (5).

8. Verfahren nach einem der vorangehenden Ansprüche, **dadurch gekennzeichnet, dass** das Anordnen der zweiten Substratschicht (3') und der zweiten Isolierschicht (5') aufweist:
Anordnen der zweiten Isolierschicht (5') zumindest bereichsweise auf dem Heizelement (7) und Anordnen der zweiten Substratschicht (3') zumindest bereichsweise auf der zweiten Isolierschicht (5'); oder
Anordnen der zweiten Isolierschicht (5') zumindest bereichsweise auf der zweiten Substratschicht (3') und Anordnen der zweiten Isolierschicht (5') zumindest bereichsweise auf dem Heizelement (7).

9. Verfahren nach einem der vorangehenden Ansprüche, **dadurch gekennzeichnet, dass** das Brennen (1060) der verpressten Schichten aufweist:
Brennen der verpressten Schichten des Mehrschichtsaufbaus bei mindestens 1400°C für mindestens 36 Stunden.

10. Verfahren nach einem der vorangehenden Ansprüche, aufweisend:
Vereinzeln des Heizers (1) vor dem Brennen (1060), insbesondere durch Ausstanzen oder durch ein Laserschneidverfahren, oder
Vereinzeln des Heizers (1) nach dem Verpressen (1050) der Schichten und vor dem Brennen (1060).

11. Verfahren nach einem der vorangehenden Ansprüche, aufweisend:
Einbringen von Ausnehmungen in die zweite Isolierschicht (5') und/oder in die zweite Substratschicht (3'), insbesondere Einbringen von Ausnehmungen zur zumindest bereichsweisen Freilegung zumindest eines Anschlussmittels des Heizelements (7).

12. Verfahren nach einem der vorangehenden Ansprüche, aufweisend:
Anordnen zumindest eines Temperatursensors (9), vor dem Verpressen der Schichten, zwischen:
(i) der ersten Isolierschicht (5) und der ersten Substratschicht (3) und/oder
(ii) der zweiten Isolierschicht (5') und der zweiten Substratschicht (3') und/oder
(iii) der ersten Isolierschicht (5) und der zweiten Isolierschicht (5').

13. Verwendung eines Heizers (1) hergestellt durch ein Verfahren (1000) nach einem der Ansprüche 1 bis 12 in einem System zur Bereitstellung eines inhalierbaren Aerosols.

14. Ein System zur Bereitstellung eines inhalierbaren Aerosols, aufweisend zumindest einen Heizer (1) hergestellt durch ein Verfahren (1000) nach einem der Ansprüche 1 bis 12.

15. Ein Heizer (1) mit einem co-gesinterten Mehrschichtaufbau für ein System zur Bereitstellung eines inhalierbaren Aerosols, aufweisend:
zumindest ein Heizelement (7) mit einer ersten Seite und einer der ersten Seite gegenüberliegenden zweiten Seite;
zumindest eine erste und eine zweite Isolierschicht (5, 5'), wobei die erste Isolierschicht (5) zumindest bereichsweise auf der ersten Seite des Heizelements (7) angeordnet ist, und wobei die zweite Isolierschicht (5') zumindest bereichsweise auf der zweiten Seite des Heizelements (7) angeordnet ist; und
zumindest eine erste Substratschicht (3) und eine zweite Substratschicht (3'), wobei die erste Substratschicht (3) zumindest bereichsweise auf der ersten Isolierschicht (5) angeordnet ist, und wobei die zweite Substratschicht (3') zumindest bereichsweise auf der zweiten Isolierschicht (5') angeordnet ist.

## Claims

1. A method for producing a heater (1) comprising a co-sintered multi-layer structure for a system for providing an inhalable aerosol, having the steps:
providing (1010) at least one first substrate layer (3);
arranging (1020) at least one first insulating layer (5) at least in regions on the first substrate layer (3);
arranging (1030) at least one heating element (7) at least in regions on the first insulating layer (5);
arranging (1040) at least one second substrate layer (3') and at least one second insulating layer (5') at least in regions on the heating element (7), wherein the second insulating layer (5') is arranged at least in regions on the second substrate layer (3'), and wherein the second insulating layer (5') is connected at least in regions to the heating element (7) and/or the first insulating layer (5);
pressing (1050) the layers and the heating element (7); and
firing (1060) the pressed layers in order to co-sinter the layers of the multi-layer structure.

2. The method according to claim 1, **characterised in that** the first substrate layer (3) and/or the second substrate layer (3') comprises/comprise at least one green film, in particular a green film having stabilised zirconium oxide, ZrO2.

3. The method according to claim 2, **characterised in that** the green film has a thickness in a range of between 0.25 mm and 0.5 mm.

4. The method according to any one of the preceding claims, **characterised in that** the first insulating layer (5) and/or the second insulating layer (5') has an aluminium oxide, Al2O3, in particular a ceramic slurry having aluminium oxide, Al2O3.

5. The method according to any one of the preceding claims, **characterised in that** the arranging of the first insulating layer (5) and/or of the second insulating layer (5') has:
screen printing and/or tape casting of the first insulating layer (5) on the first substrate layer (3) and/or of the second insulating layer (5') on the second substrate layer (3').

6. The method according to any one of the preceding claims, **characterised in that** the heating element (7) comprises at least one resistive element, in particular a resistive element comprising platinum, Pt, cermet.

7. The method according to claim 6, **characterised in that** the arranging (1030) of the heating element (7) has:
screen printing of the resistive element on the first insulating layer (5).

8. The method according to any one of the preceding claims, **characterised in that** the arranging of the second substrate layer (3') and of the second insulating layer (5') has:
arranging the second insulating layer (5') at least in regions on the heating element (7) and arranging the second substrate layer (3') at least in regions on the second insulating layer (5'); or
arranging the second insulating layer (5') at least in regions on the second substrate layer (3') and arranging the second insulating layer (5') at least in regions on the heating element (7).

9. The method according to any one of the preceding claims, **characterised in that** the firing (1060) of the pressed layers has:
firing the pressed layers of the multi-layer structure at at least 1400°C for at least 36 hours.

10. The method according to any one of the preceding claims, having:
separating the heater (1) prior to the firing (1060), in particular by means of punching or by means of a laser cutting method, or
separating the heater (1) after the pressing (1050) of the layers and prior to the firing (1060).

11. The method according to any one of the preceding claims, having:
introducing recesses into the second insulating layer (5') and/or into the second substrate layer (3'), in particular introducing recesses for exposing at least one connecting means of the heating element (7) at least in regions.

12. The method according to any one of the preceding claims, having:
arranging at least one temperature sensor (9), prior to the pressing of the layers, between:
(i) the first insulating layer (5) and the first substrate layer (3) and/or
(ii) the second insulating layer (5') and the second substrate layer (3') and/or
(iii) the first insulating layer (5) and the second insulating layer (5').

13. Use of a heater (1) produced by means of a method (1000) according to any one of claims 1 to 12 in a system for providing an inhalable aerosol.

14. A system for providing an inhalable aerosol, having at least one heater (1) produced by means of a method (1000) according to any one of claims 1 to 12.

15. A heater (1) comprising a co-sintered multi-layer structure for a system for providing an inhalable aerosol, having:
at least one heating element (7) comprising a first side and a second side located opposite the first side;
at least a first and a second insulating layer (5, 5'), wherein the first insulating layer (5) is arranged at least in regions on the first side of the heating element (7), and wherein the second insulating layer (5') is arranged at least in regions on the second side of the heating element (7); and
at least a first substrate layer (3) and a second substrate layer (3'), wherein the first substrate layer (3) is arranged at least in regions on the first insulating layer (5), and wherein the second substrate layer (3') is arranged at least in regions on the second insulating layer (5').

## Revendications

1. Procédé pour la fabrication d'un dispositif de chauffage (1) avec une structure multi-couches co-frittée pour un système destiné à la mise à disposition d'un aérosol inhalable, présentant les étapes suivantes :
mise à disposition (1010) au moins d'une première couche de substrat (3) ;
disposition (1020) au moins d'une première couche isolante (5) au moins par zones sur la première couche de substrat (3) ;
disposition (1030) au moins d'un élément chauffant (7) au moins par zones sur la première couche isolante (5) ;
disposition (1040) au moins d'une deuxième couche de substrat (3') et au moins d'une deuxième couche isolante (5') au moins par zones sur l'élément chauffant (7), la deuxième couche isolante (5') étant disposée au moins par zones sur la deuxième couche de substrat (3') et la deuxième couche isolante (5') étant en relation au moins par zones avec l'élément chauffant (7) et/ou la première couche isolante (5) ;
compression (1050) des couches et de l'élément chauffant (7) ; et
cuisson (1060) des couches comprimées, pour le co-frittage des couches de la structure multi-couches.

2. Procédé selon la revendication 1, **caractérisé en ce que** la première couche de substrat (3) et/ou la deuxième couche de substrat (3') englobe/englobent au moins une feuille verte, notamment une feuille verte présentant de l'oxyde de zirconium stabilisé, ZrO2.

3. Procédé selon la revendication 2, **caractérisé en ce que** la feuille verte présente une épaisseur comprise entre 0,25 mm et 0,5 mm.

4. Procédé selon une des revendications précédentes, **caractérisé en ce que** la première couche isolante (5) et/ou la deuxième couche isolante (5') présente un oxyde d'aluminium Al2O3, notamment une barbotine céramique présentant de l'oxyde d'aluminium, Al2O3.

5. Procédé selon une des revendications précédentes, **caractérisé en ce que** la disposition de la première couche isolante (5) et/ou de la deuxième couche isolante (5') présente :
une sérigraphie et/ou un coulage en bande de la première couche isolante (5) sur la première couche de substrat (3) et/ou de la deuxième couche isolante (5') sur la deuxième couche de substrat (3').

6. Procédé selon une des revendications précédentes, **caractérisé en ce que** l'élément chauffant (7) comprend au moins un élément de résistance, notamment un élément de résistance comprenant du platine, Pt, cermet.

7. Procédé selon la revendication 6, **caractérisé en ce que** la disposition (1030) de l'élément chauffant (7) présente :
une sérigraphie de l'élément de résistance sur la première couche isolante (5).

8. Procédé selon une des revendications précédentes, **caractérisé en ce que** la disposition de la deuxième couche de substrat (3') et de la deuxième couche isolante (5') présente les étapes suivantes :
disposition de la deuxième couche isolante (5') au moins par zones sur l'élément chauffant (7) et disposition de la deuxième couche de substrat (3') au moins par zones sur la deuxième couche isolante (5') ; ou
disposition de la deuxième couche isolante (5') au moins par zones sur la deuxième couche de substrat (3') et disposition de la deuxième couche isolante (5') au moins par zones sur l'élément chauffant (7).

9. Procédé selon une des revendications précédentes, **caractérisé en ce que** la cuisson (1060) des couches comprimées présente l'étape suivante :
cuisson des couches comprimées de la structure multi-couches à 1400°C minimum pendant au moins 36 heures.

10. Procédé selon une des revendications précédentes, présentant les étapes suivantes :
séparation du dispositif de chauffage (1) avant la cuisson (1060), notamment par estampage ou par un procédé de découpe laser ou
séparation du dispositif de chauffage (1) après la compression (1050) des couches et avant la cuisson (1060).

11. Procédé selon une des revendications précédentes, présentant l'étape suivante :
réalisation d'évidements dans la deuxième couche isolante (5') et/ou dans la deuxième couche de substrat (3'), notamment réalisation d'évidements pour une mise à nu au moins partielle au moins d'un moyen de jonction de l'élément chauffant (7).

12. Procédé selon une des revendications précédentes, présentant l'étape suivante :
disposition au moins d'une sonde de température (9) avant la compression des couches, entre :
(i) la première couche isolante (5) et la première couche de substrat (3) et/ou
(ii) la deuxième couche isolante (5') et la deuxième couche de substrat (3') et/ou
(iii) la première couche isolante (5) et la deuxième couche isolante (5').

13. Utilisation d'un dispositif de chauffage (1) fabriqué par un procédé (1000) selon une des revendications 1 à 12 dans un système destiné à la mise à disposition d'un aérosol inhalable.

14. Un système destiné à la mise à disposition d'un aérosol inhalable, présentant au moins un dispositif de chauffage (1) fabriqué par un procédé (1000) selon une des revendications 1 à 12.

15. Un dispositif de chauffage (1) avec une structure multi-couches co-frittée pour un système destiné à la mise à disposition d'un aérosol inhalable, présentant :
au moins un élément chauffant (7) avec un premier côté et un deuxième côté opposé au premier côté ;
au moins une première et une deuxième couche isolante (5, 5'), la première couche isolante (5) étant disposée au moins par zones sur le premier côté de l'élément chauffant (7) et la deuxième couche isolante (5') étant disposée au moins par zones sur le deuxième côté de l'élément chauffant (7) ; et
au moins une première couche de substrat (3) et une deuxième couche de substrat (3'), la première couche de substrat (3) étant disposée au moins par zones sur la première couche isolante (5) et la deuxième couche de substrat (3') étant disposée au moins par zones sur la deuxième couche isolante (5').
